# EUROPÄISCHE PATENTSCHRIFT

(11) **EP 1 250 299 B1**
(45) Veröffentlichungstag und Bekanntmachung des Hinweises auf die Patenterteilung: **06.04.2005**
(21) Anmeldenummer: 01925322.8
(22) Anmeldetag: 29.01.2001
(51) Int. Cl.: C07B 57/00, C07C 215/90, C07D 263/32

(54) **CHIRALE IONISCHE FLÜSSIGKEITEN**
CHIRAL IONIC LIQUIDS
LIQUIDES IONIQUES CHIRAUX

(30) Priorität: 28.01.2000 DE 10003708
(43) Veröffentlichungstag der Anmeldung: 23.10.2002
(73) Patentinhaber: Solvent Innovation GmbH, 50829 Köln (DE)
(72) Erfinder: WASSERSCHEID, Peter, 50829 Köln (DE); KEIM, Wilhelm, 52074 Aachen (DE); BOLM, Carsten, 52072 Aachen (DE); BÖSMANN, Andreas, 52068 Aachen (DE)
(74) Vertreter: Weber, Thomas, Dr.Dipl.-Chem.
(86) Internationale Anmeldenummer: PCT/EP2001/000924
(87) Internationale Veröffentlichungsnummer: WO 2001/055060

(56) Entgegenhaltungen:
- HOWARTH J ET AL: "Moisture Stable Dialkylimidazolium Salts as Heterogeneous and Homogeneous Lewis Acids in the Diels-Alder Reaction" TETRAHEDRON LETTERS, ELSEVIER SCIENCE PUBLISHERS, AMSTERDAM, NL, Bd. 38, Nr. 17, 28. April 1997 (1997-04-28), Seiten 3097-3100, XP004059107 ISSN: 0040-4039 in der Anmeldung erwähnt

## Beschreibung

Die Erfindung betrifft chirale ionische Flüssigkeiten, ein Verfahren zu deren Herstellung sowie deren Verwendung in Verfahren zur asymmetrischen Synthese, der asymmetrischen Katalyse und zur Trennung von Racematen.

Unter ionischen Flüssigkeiten versteht man allgemein Salze oder Gemische aus Salzen, deren Schmelzpunkte unterhalb 80°C liegen. Diese Salze bestehen aus Anionen wie z.B. Halogenostannaten, Halogenoaluminaten, Hexafluorophosphaten oder Tetrafluoroboraten kombiniert mit substituierten Ammonium-, Phosphonium, Pyridinium- oder Imidazolium-Kationen. Mehrere Veröffentlichungen beschreiben bereits die Verwendung ionischer Flüssigkeiten als Lösungsmittel für Übergangsmetall-katalysierte Reaktionen (T. Welton, Chem. Rev. 1999, 99, 2071; J.D. Holbry, K.R. Seddon, Clean Products and Processes, 1999, 223). Beispielsweise wurden Hydrierungen von Olefinen mit Rhodium(I) (P. A. Z. Suarez, J. E. L. Dullius, S. Einloft, R. F. de Souza und J. Dupont, Polyhedron 15/7, 1996, 1217-1219), Ruthenium(II)- und Cobalt(II)-Komplexen (P. A. Z. Suarez, J. E. L. Dullius, S. Einloft, R. F. de Souza und J. Dupont, Inorganica Chimica Acta 255, 1997, 207-209) in ionischen Flüssigkeiten mit Tetrafluoroborat-Anion erfolgreich durchgeführt. Auch die Hydroformylierung von funktionalisierten und unfunktionalisierten Olefinen mit Rhodium-Katalysatoren in ionischen Flüssigkeiten mit schwach koordinierenden Anionen (z.B. PF₆⁻, BF₄⁻) ist beschrieben (EP-A-0776880, Y. Chauvin, L. Mussmann, H. Olivier, Angew. Chem., Int. Ed. Engl., 1995, 34, 2698; W. Keim, D. Vogt, H. Waffenschmidt, P. Wasserscheid, J. of Cat., 1999, 186, 481).
Zur Synthese von binären ionischen Flüssigkeiten vom Typ [A]⁺[Y]⁻ kann ein zweistufiges Verfahren angewendet werden (J. S. Wilkes, M. J. Zaworotko, J. Chem. Soc., Chem. Commun., 13, 1992, 965). Dabei wird zunächst durch Reaktion eines Alkylierungsreagenz RX und eines Amins. NR¹R²R³ oder eines Phosphans PR¹R²R³ in einer Quarternisierungsreaktion das organische Ammoniumsalz [NR¹R²R³R]⁺ X⁻ oder das organische Phosphoniumsalz [PR¹R²R³R]⁺ X⁻ aufgebaut. X' ist dabei in der Regel ein Halogenidion. Das organische Halogenidsalz wird isoliert und in einer nachfolgenden, zweiten Reaktionsstufe in einer Austauschreaktion mit dem Alkali- oder Erdalkalisalz des Typs M⁺[Y]⁻ umgesetzt. Dies geschieht in einem Lösungsmittel in dem das Nebenprodukt M⁺ X⁻ schwerlöslich, die zu synthetisierende ionische Flüssigkeit [A]⁺[Y]⁻ dagegen gut löslich ist.
Dieses zweistufige Verfahren wurde in der Literatur zur Darstellung von ionischen Flüssigkeiten mit [BF₄]⁻-, [PF₆]⁻-, Acetat-, Nitrat-, HSO₄⁻-, SO₄²⁻-Ionen erfolgreich verwendet (J. S. Wilkes, M. J. Zaorotko, J. Chem. Soc., Chem. Commun., 13, 1992, 965, B. Ellis, WO 9618459 A1 960620, 1996 J. Fuller, R. T. Carlin, H. C. de Long, D. Haworth, J. Chem. Soc., Chem. Commun., 3, 1994, 299).
Ein einstufiges Verfahren zur Herstellung von ionischen Flüssigkeiten ist in der europäischen Patentanmeldung EP 00118442.3, ein Verfahren zur halogenidfreien Herstellung ist in der europäischen Patentanmeldung EP 00118441.5 beschrieben.
Zu ionischen Flüssigkeiten, deren Eigenschaften und Herstellung kann ferner auf P. Wasserscheid in Nachrichten aus der Chemie, 2001 (49), Seiten 12 - 16 und P. Wasserscheid und W. Keim in Angewandte Chemie, 2000 (112), 3926 - 3945 verwiesen werden.
Die bisher in ionischen Flüssigkeiten verwendeten Kationen besitzen jedoch mit Ausnahme von einem Fall keine chiralen Zentren. Die einzige ionische Flüssigkeit mit Chiralität im Kation [N,N-di-(2'S-2'-methanbutan) imidazolizumbromid] wurde von Howarth et al. beschrieben (J. Howarth, K. Hanlon, D. Fayne, P. McCormac, Tetrahedron Letters 1997, 17, 3097-3100). Diese chirale ionische Flüssigkeit besitzt ein Imidazoliumion mit einer chiralen Seitenkette und wurde bei der katalysierten asymmetrischen Diels-Alder-Reaktion verwendet, was jedoch nur zu geringen Enantiomerenüberschüssen führte. Diese Klasse chiraler ionischer Flüssigkeiten ist nur sehr aufwendig und teuer enantiomerenrein oder enantiomerenangereichert zu synthetisieren, da als Vorstufe eine enantiomerenreine oder eine enantiomerenangereicherte Chlorverbindung oder ein anderes enantiomerenreines oder ein enantiomerenangereichertes Alkylierungsmittel eingesetzt werden muss.

Aufgabe der vorliegenden Erfindung war die Zurverfügungstellung ionischer Flüssigkeiten, welche in Verfahren zur asymmetrischen Synthese, bei der asymmetrischen Katalyse sowie bei der Racemattrennung eingesetzt werden können.

In einem weiteren Aspekt der Erfindung sollte ein Verfahren zur Synthese von chiralen ionischen Flüssigkeiten zur Verfügung gestellt werden, welches gegenüber dem Stand der Technik verbessert ist.

In einem ersten Aspekt betrifft die Erfindung chirale ionische Flüssigkeiten der allgemeinen Formel

[A]ₙ⁺ [Y]ⁿ⁻,

wobei n = 1 oder 2 ist,
das Anion [Y]ⁿ⁻ das Anion einer organischen oder anorganischen Protonensäure ist und
das Kation [A]⁺ ein optisch aktives organisches Ammonium-Kation mit bis zu 50 Kohlenstoffatomen ist, wobei das Ammonium-Kation [A]⁺ die allgemeine Formel

[NR^{w}R^{x}R^{y}R^{z}]⁺

besitzt, und
R^{w}, R^{x}, R^{y} und R^{z} unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 30 Kohlenstoffatomen;
- Heteroaryl-, Heteroaryl-C₁-C₆-Alkylgruppen mit 3 bis 8 Kohlenstoffatoman im Heteroaryl-Rest und wenigstens einem Heteroatom ausgewählt aus N, O und S, die mit wenigstens einer Gruppe ausgewählt aus C₁-C₆-Alkylgruppen und/oder Halogenatomen substituiert sein kann;
- Aryl-, Aryl-C₁-C₆-Alkylgruppen mit 5 bis 12 Kohlenstoffatomen; und
- Silyl-Gruppen der allgemeinen Formel -SiR^{t}R^{u}R^{v}, wobei R^{t}, R^{u} und R^{v} unabhängig voneinander ausgewählt sind aus der Gruppe bestehend aus C₁-C₆-Alkyl und C₅-C₁₂-Aryl;
wobei wenigstens eine dieser Gruppen R^{w}, R^{x}, R^{y} und R^{z} wenigstens ein Chiralitätszentrum aufweist;
wenigstens eine der Gruppen R^{w}, R^{x}, R^{y} und R^{z} mit wenigstens einer funktioneilen Gruppe entweder substituiert ist oder diese enthält und diese funktionelle Gruppe ausgewählt ist aus der Gruppe bestehend aus -OH, Ether- (-OR), Thiol- (-SH), Thioether- (-SR), Nitril- (-CN), Carbonsäure- (-COOH), Carbonsäureester- (-COOR), Phosphan- (-PR₂), Keton- (-COR), Aldehyd- (-CHO), Nitro- (-NO₂), Azid- (-N₃), Phenyl-, Fluorid- oder Chtorid-, wobei R ausgewählt ist aus der Gruppe bestehend aus
- Wasserstoff;
- linearen oder verzweigten, gesättigten oder ungesättigten, aliphatischen oder alicyclischen Alkylgruppen mit 1 bis 30 Kohlenstoffatomen, wobei die linearen oder verzweigten aliphatischen Alkylgruppen mit einer Hydroxylgruppe substituiert sein können;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein kann;
- Aryl-, Aryl-C₁-C₆-Alkylgruppen mit 5 bis 12 Kohlenstoffatomen im Arylrest, der gegebenenfalls mit wenigstens einer C₁-C₆-Alkylgruppe und/oder einem Halogenatom substituiert sein kann, und
- Silyl-Gruppen der allgemeinen Formel -SiR⁶R⁷R⁸, wobei R⁶, R⁷ und R⁸ unabhängig voneinander ausgewählt sind aus der Gruppe bestehend aus C₁-C₆-Alkyl und C₅-C₁₂-Aryl;
zwei der Gruppen R^{w}, R^{x}, R^{y} und R^{z} unter Bildung eines 4, 5, 6 oder 7-gliedrigen gesättigten oder ungesättigten Ringes, der zusätzlich wenigstens ein Heteroatom, ausgewählt aus Stickstoff, Sauerstoff oder Schwefel enthalten kann, verknüpft sein können, und dieser Ring mit wenigstens einer der obengenannten Alkyl-, Aryl- oder Heteroaryl-Gruppen substituiert sein kann;
wobei wenigstens ein Chiralitätszentrum einen Abstand von bis zu 5 Atombindungen von der funktionellen Gruppe hat;
mit der Maßgabe, dass nicht mehr als ein, vorzugsweise nicht mehr als zwei, besonders bevorzugt nicht mehr als drei der Gruppen R^{w}, R^{x}, R^{y} und R^{z} gleichzeitig Wasserstoff sind.

Die erfindungsgemäßen ehiralen ionischen Flüssigkeiten weisen einen Schmelzpunkt von bis zu 100°C und vorzugsweise bis zu 80°C auf.
Als Protonensäure deren Anion das Anion [Y]ⁿ⁻ bilden kann, kommt grundsätzlich jede Protonensäure in Frage, insbesondere jedoch solche mit einer Säurekonstante pkₛ ≤13, vorzugsweise mit einer Säurekonstante pkₛ ≤ 8, besonders bevorzugt mit einer Säurekonstante pkₛ≤5.
Die erfindungsgemäßen chiralen ionischen Flüssigkeiten können auch aus Gemischen der genannten Kationen und Anionen bestehen, insbesondere aus einem . Gemisch von wenigstens zwei unterschiedlichen Kationen und einem Anion, einem Kation und wenigstens zwei unterschiedlichen Anionen oder wenigstens zwei unterschiedlichen Anionen und wenigstens zwei unterschiedlichen Anionen bestehen.

In einer Ausführungsform sind die erfindungsgemäßen chiralen ionischen Flüssigkeiten dadurch gekennzeichnet, dass das Anion [Y]ⁿ⁻ der organischen oder anorganischen Protonensäure ausgewählt ist aus der Gruppe bestehend aus, Fluorid, Chlorid, Bromid, Iodid, Nitrat, HSO₄⁻, H₂PO₄⁻, HPO₄²⁻, Bis-(trifluormethansulfon)-imidat, Tetrafluoroborat ([BF₄]⁻), Tetrachloroborat ([BCl₄]⁻), Hexafluorophosphat ([PF₆]⁻), Hexafluoroantimonat ([SbF₆]⁻), Hexafluoroarsenat ([AsF₆]⁻), Tetrachloroaluminat ([AlCl₄]⁻), Trichlorozinkat [(ZnCl₃]⁻), Dichlorocuprat, Sulfat ([SO₄]²⁻), Carbonat ([CO₃]²⁻), Fluorosulfonat, [R'-COO]⁻, [R'-SO₃]⁻ oder [(R'-SO₂)₂N]⁻, wobei R' ein linearer oder verzweigter 1 bis 12 Kohlenstoffatome enthältender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-aryl-Rest ist, der durch Halogenatome, insbesondere durch Fluor und Chlor substituiert sein kann. Die Gruppe R' kann wenigstens ein Chiralitätszentrum aufweisen. Hierzu ist dann von entsprechenden optisch aktiven Ausgangsverbindungen auszugehen. Als Beispiele von Anionen der allgemeinen Formel R'-COO⁻ seien Butanoat-, Hexanoat-, Citrat-, Tartrat-, Lactat- oder Succinat-Anionen genannt.

Das wenigstens ein Chiralitätszentrum enthaltende chirale (optisch aktive) Ammonium-Kation [A]⁺ weist vorzugsweise bis zu 40, besonders bevorzugt bis zu 25 Kohlenstoffatome, vorzugsweise wenigstens 3, besonders bevorzug wenigsteris 5 Kohlenstoffatome auf.
Ferner weist das Kation [A]⁺ vorzugsweise eine Alkohol- (OH), Ether- (OR), Thiol- (SH), Thioether- (SR), Nitril- (CN), Carbonsäure- (COOH), Carbonsäureester- (COOR), Phosphan- (PR₂), Keton- (COR), Aldehyd- (CHO), Nitro- (NO₂), Azid- (N₃), Phenyl-, Fluorid- oder Chlorid-Gruppe als funktionelle Gruppe G auf. Diese Gruppen sind durch Ausbildung von Wasserstoffbrücken oder durch Zurverfügungstellung von freien Elektronenpaaren zur einer Koordination mit anderen Molekülen, weiche Wasserstoffbrücken-Acceptoren und/oder Acceptoren für freie Elektronenpaare aufweisen, befähigt. Wenigstens ein Chiralitätszentrum weist einen Abstand von 1 bis 5 Atombindungen, vorzugsweise bis zu drei Atombindungen von der funktionellen Gruppe auf.

In einer besonders bevorzugten Ausgestaltung sind die erfindungsgemäßen chiralen ionischen Flüssigkeiten dadurch gekennzeichnet, dass [A]⁺ ausgewählt ist aus der Gruppe bestehend aus 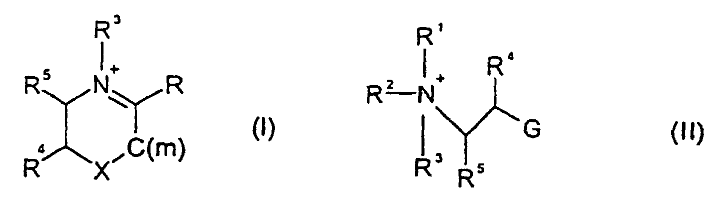 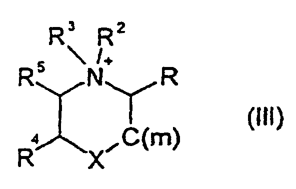 wobei m = 0 oder 1 ist,
X aus gewählt ist aus der Gruppe bestehend aus Stickstoff, Sauerstoff und Schwefel,
R, R¹, R², R³, R⁴ und R⁵ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 30 Kohlenstoffatomen, wobei die linearen oder verzweigten aliphatischen Alkylgruppen mit einer Hydroxyl- oder Thiolgruppe substituiert sein können;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein kann;
- Aryl-, Aryl-C₁-C₆-Alkylgruppen mit 5 bis 12 Kohlenstoffatomen im Arylrest, der gegebenenfalls mit wenigstens einer C₁-C₆-Alkylgruppe und/oder einem Halogenatom substituiert sein kann, und
- Silyl-Gruppen der allgemeinen Formel -SiR⁶R⁷R⁸, wobei R⁶, R⁷ und R⁸ unabhängig voneinander ausgewählt sind aus der Gruppe bestehend aus C₁-C₆-Alkyl und C₅-C₁₂-Aryl, vorzugsweise C₁-C₃-Alkyl, sowie
G ausgewählt ist aus der Gruppe bestehend aus -OH, Ether- (-OR), Thiol-, Thioether- (-SR), Nitril- (-CN), Carbonsäure- (-COOH), Carbonsäureester- (-COOR), Phosphan- (-PR₂), Keton- (-COR), Aldehyd- (-CHO), Nitro- (-NO₂), Azid- (-N₃), Phenyl-, Fluorid- oder Chlorid-, wobei R die obengenannten Bedeutungen hat, mit der Maßgabe, dass R⁴ und R⁵ nicht gleichzeitig Wasserstoff sind.

Die linearen oder verzweigten, gesättigten oder ungesättigten, aliphatischen oder alicyclischen Alkylgruppen weisen vorzugsweise bis zu 20 Kohlenstoffatome auf, besonders bevorzugt bis zu 8 Kohlenstoffatome und sind beispielhaft ausgewählt aus der Gruppe bestehend aus der Methyl-, Ethyl-, Propyl-, iso-Propyl-, n-, iso-, sek.-, tert.-Butyl-, den verschiedenen Isomeren der Pentyl-, Hexyl-, Heptyl- und Octyl-Gruppen, die gegebenenfalls mit einer Hydroxylgruppe substituiert sein können sowie der entsprechenden ungesättigten und oder cyclischen Gruppen, sofern sie existieren. In einer bevorzugten Ausgestaltung sind R, R¹, R², R³, R⁴ und R⁵ unabhängig voneinander Wasserstoff oder die genannten Alkylgruppen.

In einer weiteren Alternative der Erfindung sind die chiralen ionischen Flüssigkeiten dadurch gekennzeichnet, dass R, R¹, R², R³, R⁴, R⁵ R⁶, R⁷ und R⁸ unabhängig voneinander wenigstens ein Chiralitätszentrum aufweisen können. Soweit nicht anders spezifiziert bedeutet "Halogenatom" oder "Halogenid" Fluor(id), Chlor(id), Brom(id) oder Iod(id), vorzugsweise Fluor, Chlor oder Brom. Mit "C₁-C₆-Alkylgruppe" sind lineare, verzweigte oder alicyclische Gruppen gemeint, insbesondere jedoch die Methyl-, Ethyl-, n-Propyl-, iso-Propyl-, n-Butyl-, iso-Butyl-, sek.-Butyl- oder tert.-Butyl-Gruppe. Unter "C₅-C₁₂-Aryl-Gruppe" sind insbesondere die Phenyl- oder die Naphthylgruppe zu verstehen.

In einem weiteren Aspekt betrifft die Erfindung ein Verfahren zur Herstellung der oben genannten chiralen ionischen Flüssigkeiten. Das erfindungsgemäße Verfahren erlaubt einen wesentlich einfacheren und kostengünstigeren Zugang zu ionischen Flüssigkeiten mit chiralen Kationen. Dies wird ermöglicht durch ein neues Konzept zur Synthese chiraler ionischer Flüssigkeiten. Dabei wird nicht wie bei den von Howarth et al veröffentlichten chiralen ionischen Flüssigkeiten die Chiralität im Alkylierungsschritt in das Kation eingeführt. Auch werden nicht wie bisher die allgemein zur Synthese ionischer Flüssigkeiten üblichen Ammonium-, Phosphonium-, Pyridinium- oder Imidazolium-Kationen verwendet.

Im einzelnen betrifft die Erfindung ein Verfahren zur Herstellung der oben genannten chiralen ionischen Flüssigkeiten durch Alkylierung eines dem Ammonium-Kation [A]⁺ der allgemeinen Formel [NR^{w}R^{x}R^{y}R^{z}]⁺ zugrunde liegenden Amines NR^{x}R^{y}R^{z} mit mit einem Alkylierungsreagenz der Formel R^{w}X¹, R^{w}SO₄R^{w}, R'SO₃R^{w} oder [R^{w}₃O]⁺BF₄⁻, wobei X¹ Fluorid, Chlorid, Bromid oder Iodid ist, R^{w} die oben angegebene Bedeutung hat, jedoch nicht Wasserstoff ist, R^{x}, R^{y} und R^{z} die oben angegebenen Bedeutungen haben,
mit der Maßgabe, dass nicht mehr als zwei, vorzugsweise nicht mehr als eine der Gruppen R^{x}, R^{y} und R^{z} gleichzeitig Wasserstoff sind.
Der oben verwendete Begriff "Alkylierung" ist sinngemäß durch "(Hetero-) Arylierung" oder "Silylierung" zu ersetzen, wenn entsprechende, oben erwähnte Gruppen eingeführt werden.

In einer besonderen Ausgestaltung einzelnen betrifft die Erfindung ein Verfahren zur Herstellung der oben genannten chiralen ionischen Flüssigkeiten durch Umsetzung [Alkylierung, (Hetero-)Arylierung oder Silylierung] der optisch aktiven Amine 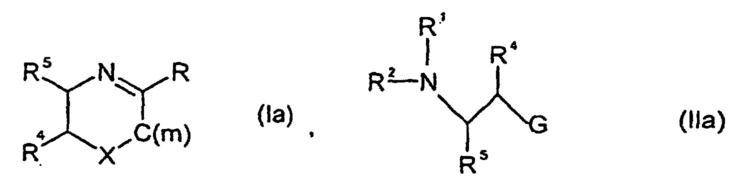 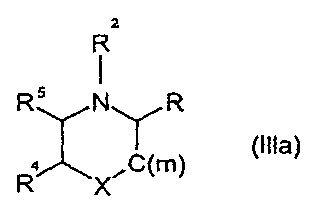 mit einem Reagenz [Alkylierungsreagenz, (Hetero-)Arylierungsreagenz, Silylierungsreagenz) der Formel R³X¹, R³SO₄R^{3'}, R'SO₃R³ oder [R³₃O]⁺BF₄⁻, wobei X¹ Fluorid, Chlorid, Bromid oder Iodid ist, R' die oben angegebene Bedeutung hat, R³ die oben angegebene Bedeutung hat und R^{3'} aus der Gruppe von R³ ausgewählt ist, jedoch von R³ verschieden sein kann. Bevorzugt ist jedoch R³ = R^{3'}. Vorzugsweise werden Di-R³-sulfate der allgemeinen Formel R³-SO₄-R³ eingesetzt, also symmetrische Organodisulfate in welcher R³ die obengenannte Bedeutung hat. Bevorzugt sind Di-C₁-C₆-Alkylsulfate, insbesondere Dimethyl-, Diethyl-, Di-npropyl-, Diisopropyl-, Di-n-butyl-, Diisobutyl-, Di-tert.-butyl-, Di-n-pentyl-, Diisopentyl-, Di-neo-pentyl-, Di-n-hexylsulfat sowie Dicyclohexylsulfat. Ferner bevorzugt sind C₁-C₆-Alkyliodide und -Bromide sowie die [(C₁-C₆-)₃O]⁺BF₄⁻ -Meerwein Salze.
In einem nachfolgenden Reaktionsschritt kann das Anion X¹⁻, R^{3'}SO₄⁻, R'SO₃⁻ und BF₄⁻ durch literaturbekannte Austauschreaktionen in ein davon verschiedenes Anion [Y]⁻ oder [Y]²⁻ überführt werden.
Die optisch aktiven Amine Ia, IIa und IIIa sind im Stand der Technik bekannt, teilweise im Handel erhältlich oder ohne weiteres durch bekannte Syntheseverfahren herstellbar. Einfach Ausgangsprodukte sind beispielsweise Verbindungen, die sich von Aminosäuren ableiten, Oxazoline, Dihydroimidazoline, Thiazoline, Dihydrothiazoline, Nicotin, Ephedrin, 2-Hydroxyalkylamine, 2-Alkoxyalkylamine, etc.. Die optische Reinheit der erfindungsgemäßen ionischen Flüssigkeiten ist im wesentlichen von der optischen Reinheit der chiralen Ausgangsverbindungen abhängig.
Im Gegensatz zu den von Howarth et al beschriebenen chiralen ionischen Flüssigkeit wird erfindungsgemäß zur Synthese der chiralen ionischen Flüssigkeiten in enantiomerenreiner oder enantiomerenangereicherter Form kein teures enantiomerenreines oder enantiomerenangereichertes Alkylierungsreagenz benötigt. Vielmehr sind erfindungsgemäß alle Kationen durch einfache, literaturbekannte organische Syntheseschritte direkt aus natürlich verfügbaren, enantiomerenreinen Ausgangsstoffen in enantiomerenreiner oder enantiomerenangereicherter Form zugänglich ("chiral pool"). Dadurch sind die neuartigen chiralen ionischen Flüssigkeiten in großen Mengen enantiomerenrein oder enantiomerenangereichert preiswert zugänglich.

Die Reaktion wird bei Temperaturen von -196 °C bis +150 °C, vorzugsweise zwischen-80 °C und +80 °C, ganz besonders bevorzugt zwischen 0 °C und +60 °C durchgeführt. Die Herstellung und Aufarbeitung der ionischen Flüssigkeiten kann in dafür geeigneten Lösungsmitteln oder in Substanz erfolgen.
Die Verfahren zur Herstellung ionischer Flüssigkeiten einschließlich des Anionenaustausches sind von den einzelnen Systemen abhängig und im Stand der Technik bekannt. Insoweit kann auf die in der Einleitung erwähnten Literaturzitate verwiesen werden.
Die obengenannten erfindungsgemäßen chiralen ionischen Flüssigkeiten können zur Trennung von Racematen in die einzelnen Enantiomere, als Lösungsmittel für die asymmetrische anorganische und organische Synthese, als auch als Lösungsmittel für die asymmetrische Katalyse bei organischen und anorganischen Reaktionen verwendet werden.
Die Trennung der Racemate erfolgt über die Ausbildung eines zweiphasigen Systems wobei eine Phase durch das Racemat, die andere durch die chirale ionische Flüssigkeit gebildet wird. Bedingt durch die unterschiedliche Löslichkeit der Enantiomere in der chiralen ionischen Flüssigkeit kommt es zu einer Anreicherung des besser löslichen Enantiomers in der chiralen ionischen Flüssigkeit.
Alternativ kann das Racemat in einem geeigneten Lösungsmittel gelöst werden. Diese Lösung des Racemats bildet dann mit der chiralen ionischen Flüssigkeit das zweiphasige System aus. Wiederum bedingt durch die unterschiedliche Löslichkeit der Enantiomere in der chiralen ionischen Flüssigkeit kommt es zu einer Anreicherung des in der chiralen ionischen Flüssigkeit besser löslichen Enantiomers in dieser chiralen ionischen Flüssigkeit.
Dementsprechend können die chiralen ionischen Flüssigkeiten gemäß dieser Erfindung als Chlatratbildner bei der Racematspaltung oder Enantiomerentrennung durch Extraktivkristallisation oder als Lösungsmittel für die extraktive Racematspaltung oder Enantiomerentrennung verwendet werden.
Weitere Verwendungsgebiete der erfindungsgemäßen ionischen Flüssigkeiten sind in deren Verwendung als Lösungsmittel für die asymmetrische organischen und anorganischen Synthese, beispielsweise bei Diels-Alder Reaktionen und Benzoin-Reaktionen und die asymmetrische Katalyse, insbesondere bei der Hydrierung und der Hydrovinylierung zu sehen.

Dementsprechend betrifft die Erfindung auch Verfahren zur Racematspaltung und Enantiomerentrennung, zur asymmetrischen Synthese sowie zur asymmetrischen Katalyse unter Verwendung der erfindungsgemäßen chiralen ionischen Flüssigkeiten.
In Gegenwart der erfindungsgemäßen chiralen ionischen Flüssigkeiten verlaufen die Racemattrennung, die asymmetrische Synthese und die asymmetrische Katalyse unter hohen Enantiomerenausbeuten.
Die nachfolgenden Beispiele dienen der Erläuterung der Erfindung ohne sie jedoch zu beschränken.

### Beispiele

1H und 13C NMR spektroskopische Bestimmungen wurden mit einem NMR Spektrometer DPX 300 der Firma Bruker durchgeführt.
Der Enantiomerenüberschuss wurde bestimmt durch Derivatisierung der erhaltenen chiralen ionischen Flüssigkeit mit Mosher's Reagenz und anschließender NMR-spektroskopischer Auswertung der Enantiomerenpeaks.

### 1) Synthese der chiralen ionischen Flüssigkeit 4-(S)-Isopropyl-2,3-dimethyl-oxazoliniumtetrafluoroborat

117 g (1 mol) L-Valin werden mit 100 g (2,5 mol) Natriumborhydrid in 1000 ml THF suspendiert und auf 0°C gekühlt. Eine Lösung von 66 ml (1,25 mol) Schwefelsäure in 150 ml Diethylether wird unter Rühren so zugegeben, dass die Temperatur der Reaktionsmischung unterhalb 20°C bleibt. Nach beendeter Zugabe wird auf Raumtemperatur erwärmt und 12h gerührt. Zur Zerstörung von überschüssigem Diboran werden 100 ml Methanol zugegeben. Die Reaktionsmischung wird am Rotationsverdampfer auf etwa 500 ml eingeengt und 1000 ml 5N Natronlauge zugefügt. Die Lösungsmittel werden bis 100°C abdestilliert, danach wird 3h unter Rückfluss gekocht. Die Lösung wird mit Dichlormethan extrahiert, und das Dichlormethan wird im Vakuum entfernt. Die Ausbeute an Valinol beträgt 86,5 g (84%).
20 g (0.194 mol) Valinol werden mit 11,64 g (0,194 mol) Essigsäure in 60 ml Toluol gelöst und 24h unter Rückfluss am Wasserabscheider gekocht. Nach dem Abkühlen wird die Lösung mit 10% Salzsäure extrahiert. Die wässrige Phase wird mit 40% Natronlauge neutralisiert und mit Diethylether extrahiert. Die organische Phase wird über Na₂SO₄ getrocknet und das Lösungsmittel entfernt. Destillation (112°C) ergibt 19,2 g (78%) 4-(S)-Isopropyl-2-methyloxazolin als farbloses Öl.
15 g (0,12 mol) 4-(S)-Isopropyl-2-methyloxazolin werden in 50 ml Dichlormethan gelöst und auf -20°C gekühlt. 18 g (0,122 mol) Meerwein's Reagenz werden in Portionen zugegeben. Die Reaktionsmischung wird nach beendeter Zugabe 2h bei Raumtemperatur gerührt. Das Lösungsmittel wird im Vakuum entfernt und der Rückstand mit Diethylether gewaschen. Die Ausbeute an 4-(S)-Isopropyl-2,3-dimethyl-oxazoliniumtetrafluoroborat beträgt 27 g. Der Enantiomerenüberschuss im Produkt beträgt >95 %.

NMR-Daten:
¹H-NMR (CDCl₃): 5,22 (ddd, 1H); 4,69 (d, 2H); 3,53 (s, 3H); 2,41(m, 1H); 2,17 (s, 3H); 0,99 (d, 3H); 0,93 (d, 3H).
¹³C-NMR (CDCl₃): 176,4; 72,1; 68,4; 53,8; 26,7; 18,0; 15,6; 15,0.

### 2) Synthese der chiralen ionischen Flüssigkeit 3-Butyl-4-(S)-isopropyl-2-methyloxazoliniumtetrafluoroborat

117 g (1 mol) L-Valin werden mit 100 g (2,5 mol) Natriumborhydrid in 1000 ml THF suspendiert und auf 0°C gekühlt. Eine Lösung von 66 ml (1,25 mol) Schwefelsäure in 150 ml Diethylether wird unter Rühren so zugegeben, dass die Temperatur der Reaktionsmischung unterhalb 20°C bleibt. Nach beendeter Zugabe wird auf Raumtemperatur erwärmt und 12h gerührt. Zur Zerstörung von überschüssigen Diboran werden 100 ml Methanol zugegeben. Die Reaktionsmischung wird am Rotationsverdampfer auf ca. 500 ml eingeengt und 1000 ml 5N Natronlauge zugefügt. Die Lösungsmittel werden bis 100° C abdestilliert, danach wird 3h unter Rückfluss gekocht. Die Lösung wird mit Dichlormethan extrahiert. Das Dichlormethan wird im Vakuum entfernt. Die Ausbeute an Valinol beträgt 86,5 g (84%).
20 g (0,194 mol) Valinol werden mit 11,64 g (0,194 mol) Essigsäure in 60 ml Toluol gelöst und 24h unter Rückfluss am Wasserabscheider gekocht. Nach dem Abkühlen wird die Lösung mit 10% Salzsäure extrahiert. Die wässrige Phase wird mit 40% Natronlauge neutralisiert und mit Diethylether extrahiert. Die organische Phase wird über Na₂SO₄ getrocknet und das Lösungsmittel entfernt. Destillation (112°C) ergibt 19,2 g (78%) 4-(S)-Isopropyl-2-methyloxazolin als farbloses Öl.
15 g (0,12 mol) 4-(S)-Isopropyl-2-methyloxazolin werden in 50 ml Dichlormethan gelöst und bei Raumtemperatur unter Rühren mit 17,8 g (0,13 mol) Butylbromid versetzt. Nach beendeter Zugabe wird 2h auf 50°C erwärmt. Nach Entfernen des Lösungsmittels unter Vakuum erhält man 31,4 g (99%) 3-Butyl-4-(S)-isopropyl-2-methyl-oxazotiniumbromid.
Dieses wird in 50 ml Dichlormethan gelöst und mit 22 g (0,2 mol) Natriumtetrafluoroborat versetzt. Diese Mischung wird 5 Tage bei Raumtemperatur gerührt. Die Salze werden abfiltriert und das Lösungsmittel im Vakuum entfernt. Die Ausbeute an 3-Butyl-4-(S)-isopropyl-2-methyloxazolinium-tetrafiluoroborat beträgt 29,5 g. Der Enantiomerenüberschuss im Produkt beträgt >95 %.

NMR-Daten:
¹H-NMR (CDCl₃): 5,13 (ddd, 1H); 4,72 (dd, 2H); 4,42 (t, 2H); 2,43 (m, 1H); 2,22 (s, 3H); 1,99 (tt, 2H); 1,39 (tt, 2H); 1,05 (d, 3H), 0,99 (t, 3H); 0,95 (d, 3H).
¹³C-NMR (CDCl₃): 175,3; 71,0; 67,8; 54,3; 50,1; 31,4; 25,5; 19,2; 17,2; 14,8; 12,9.

### 3) Synthese der chiralen ionischen Flüssigkeit 3-Butyl-4-(S)-isopropyl-2-methyloxazoliniumhexafluorophosphat

117 g (1 mol) L-Valin werden mit 100 g (2.5 mol) Natriumborhydrid in 1000 ml THF suspendiert und auf 0°C gekühlt. Eine Lösung von 66 ml (1.25 mol) Schwefelsäure in 150 ml Diethylether wird unter Rühren so zugegeben, dass die Temperatur der Reaktionsmischung unterhalb 20°C bleibt. Nach beendeter Zugabe wird auf Raumtemperatur erwärmt und 12h gerührt. Zur Zerstörung von überschüssigen Diboran werden 100 ml Methanol zugegeben. Die Reaktionsmischung wird am Rotationsverdampfer auf ca. 500 ml eingeengt und 1000 ml 5N Natronlauge zugefügt. Die Lösungsmittel werden bis 100°C abdestilliert, danach wird 3h unter Rückfluss gekocht. Die Lösung wird mit Dichlormethan extrahiert. Das Dichlormethan wird im Vakuum entfernt. Die Ausbeute an Valinol beträgt 86,5g (84%).
20 g (0.194 mol) Valinol werden mit 11.64 g (0.194 mol) Essigsäure in 60 ml Toluol gelöst und 24h unter Rückfluss am Wasserabscheider gekocht. Nach dem Abkühlen wird die Lösung mit 10% Salzsäure extrahiert. Die wässrige Phase wird mit 40% Natronlauge neutralisiert und mit Diethylether extrahiert. Die organische Phase wird über NaSO₄ getrocknet und das Lösungsmittel entfernt. Destillation (112°C) ergibt 19.2 g (78%) 4-(S)-Isopropyl-2-methyloxazolin als farbloses Öl.
15 g (0.12 mol) des Oxazolins werden in 50 ml Dichlormethan gelöst. und bei Raumtemperatur unter Rühren mit 17.8 g (0.13 mol) Butylbromid versetzt. Nach beendeter Zugabe wird 2h auf 50°C erwärmt. Nach Entfernen des Lösungsmittels unter Vakuum erhält man 31.4 g (99%) 3-Butyl-4-(S)-isopropyl-2-methyl-oxazoliniumbromid.
20 g (0.074 mol) Oxazoliniumsalz werden in 100 ml Wasser gelöst und bei Raumtemperatur unter Rühren mit 0.1 mol Hexafluorophosphorsäure in wässriger Lösung versetzt. Die Reaktionsmischung wird mehrfach mit Wasser gewaschen und das Lösungsmittel im Vakuum entfernt. Die Ausbeute an 3-Butyl-4-(S)-isopropyl-2-methyl-oxazoliniumhexafluorophosphat beträgt 24 g. Der Enantiomerenüberschuss im Produkt beträgt >95 %.

NMR-Daten:
¹H-NMR (CDCl₃): 5,22 (ddd, 1H); 4,69 (dd, 2H); 4,40 (t, 2H); 2,41 (m, 1H); 2,17 (s, 3H); 1,93 (tt, 2H); 1,35 (tq, 2H); 0,99 (d, 3H), 0,96 (t, 3H); 0,93 (d, 3H)
¹³C-NMR (CDCl₃): 176,4; 72,1; 68,4; 53,8; 49,6; 32,1; 26,7; 19,4; 18,0; 15,0; 13,4

### 4) Synthese der chiralen ionischen Flüssigkeit (1-Hydroxymethyl-2-methl-propyl)-trimethylammonium-hexafluorophosphat

117 g (1 mol) L-Valin werden mit 100 g (2,5 mol) Natriumborhydrid in 1000 ml THF suspendiert und auf 0°C gekühlt. Eine Lösung von 66 ml (1,25 mol) Schwefelsäure in 150 ml Diethylether wird unter Rühren so zugegeben, dass die Temperatur der Reaktionsmischung unterhalb 20°C bleibt. Nach beendeter Zugabe wird auf Raumtemperatur erwärmt und 12h gerührt. Zur Zerstörung von überschüssigem Diboran werden 100 ml Methanol zugegeben. Die Reaktionsmischung wird am Rotationsverdampfer auf etwa. 500 ml eingeengt und 1000 ml 5N Natronlauge zugefügt. Die Lösungsmittel werden bis 100°C abdestilliert, danach wird 3h unter Rückfluss gekocht. Die Lösung wird mit Dichlormethan extrahiert. Das Dichlormethan wird im Vakuum entfernt. Die Ausbeute an Valinol beträgt 86,5 g (84%).
58,5 g (0,5 mol) Valinol werden in 115 g (2,5 mol) Ameisensäure gelöst und auf 0°C gekühlt. Zu dieser Mischung werden 1,2 mol Formaldehyd gegeben und bis zur Beendigung der Kohlendioxidentwicklung auf dem siedenden Wasserbad erwärmt. Die Lösung wird mit Salzsäure angesäuert und die Lösungsmittel unter Vakuum entfernt. Der Rückstand wird in 50 ml Wasser gelöst, mit 25% Natronlauge basisch gemacht und mit Diethylether extrahiert. Die Extrakte werden über Natriumsulfat getrocknet und das Lösungsmittel im Vakuum entfernt. Destillation (33mbar/80°C) ergibt 53,7 g (82%) N,N-Dimethylvalinot als farbloses Öl. 50 g (0,38 mol) N,N-Dimethylvalinol werden in 100 ml Dichlormethan gelöst, mit 56,8 g (0,4 mol) Methyliodid versetzt und bei Raumtemperatur 12h gerührt. Das Lösungsmittel wird unter Vakuum entfernt, der Rückstand in 200 ml Wasser gelöst und unter Eiskühlung mit 0,4 mol Hexafluorophosphorsäure in wässriger Lösung versetzt. Die Reaktionsmischung wird mehrfach mit Wasser gewaschen und das Lösungsmittel im Vakuum entfernt. Die Ausbeute an (1-Hydroxymethyl-2-methyl-propyl)-trimethylammonium-hexafluorophosphat beträgt 107 g. Der Enantiomerenüberschuss im Produkt beträgt >95 %.

## Patentansprüche

1. Chirale ionische Flüssigkeiten der allgemeinen Formel
[A]ₙ⁺[Y]ⁿ⁻,
wobei
n = 1 oder 2 ist,
das Anion [Y]ⁿ⁻ das Anion einer organischen oder anorganischen Protonensäure ist; und
das Kation [A]⁺ ein optisch aktives organisches Ammonium-Kation mit bis zu 50 Kohlenstoffatomen ist, wobei das Ammonium-Kation [A]⁺ die allgemeine Formel
[NR^{w}R^{x}R^{y}R^{z}]⁺
besitzt, und
R^{w}, R^{x}, R^{y} und R^{z} unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 30 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, die mit wenigstens einer Gruppe ausgewählt aus C₁-C₆-Alkylgruppen und/oder Halogenatomen substituiert sein kann;
- Aryl-, Aryl-C₁-C₆-Alkylgruppen mit 5 bis 12 Kohlenstoffatomen; und
- Silyl-Gruppen der allgemeinen Formel -SiR^{t}R^{u}R^{v}, wobei R^{t}, R^{u} und R^{v} unabhängig voneinander ausgewählt sind aus der Gruppe bestehend aus C₁-C₆-Alkyl und C₅-C₁₂-Aryl;
wobei wenigstens eine dieser Gruppen R^{w}, R^{x}, R^{y} und R^{z} wenigstens ein Chiralitätszentrum aufweist;
wenigstens eine der Gruppen R^{w}, R^{x}, R^{y} und R^{z} mit wenigstens einer funktionellen Gruppe entweder substituiert ist oder diese enthält und diese funktionelle Gruppe ausgewählt ist aus der Gruppe bestehend aus -OH, Ether- (-OR), Thiol- (-SH), Thioether- (-SR), Nitril- (-CN), Carbonsäure- (-COOH), Carbonsäureester- (-COOR), Phosphan- (-PR₂), Keton- (-COR), Aldehyd- (-CHO), Nitro- (-NO₂), Azid- (-N₃), Phenyl-, Fluorid- oder Chlorid-, wobei R ausgewählt ist aus der Gruppe bestehend aus
- Wasserstoff;
- linearen oder verzweigten, gesättigten oder ungesättigten, aliphatischen oder alicyclischen Alkylgruppen mit 1 bis 30 Kohlenstoffatomen, wobei die linearen oder verzweigten aliphatischen Alkylgruppen mit einer Hydroxylgruppe substituiert sein können;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein kann;
- Aryl-, Aryl-C₁-C₆-Alkylgruppen mit 5 bis 12 Kohlenstoffatomen im Arylrest, der gegebenenfalls mit wenigstens einer C₁-C₆-Alkylgruppe und/oder einem Halogenatom substituiert sein kann, und
- Silyl-Gruppen der allgemeinen Formel -SiR⁶R⁷R⁸, wobei R⁶, R⁷ und R⁸ unabhängig voneinander ausgewählt sind aus der Gruppe bestehend aus C₁-C₆-Alkyl und C₅-C₁₂-Aryl;
zwei der Gruppen R^{w}, R^{x}, R^{y} und R^{z} unter Bildung eines 4, 5, 6 oder 7-gliedrigen gesättigten oder ungesättigten Ringes, der zusätzlich wenigstens ein Heteroatom, ausgewählt aus Stickstoff, Sauerstoff oder Schwefel enthalten kann, verknüpft sein können, und dieser Ring mit wenigstens einer der obengenannten Alkyl-, Aryl- oder Heteroaryl-Gruppen substituiert sein kann;
wobei wenigstens ein Chiralitätszentrum einen Abstand von bis zu 5 Atombindungen von der funktionellen Gruppe hat;
mit der Maßgabe, dass nicht mehr als ein, vorzugsweise nicht mehr als zwei, besonders bevorzugt nicht mehr als drei der Gruppen R^{w}, R^{x}, R^{y} und R^{z} gleichzeitig Wasserstoff sind.

2. Chirale ionische Flüssigkeiten nach Anspruch 1, **dadurch gekennzeichnet, dass** sie aus einem Gemisch von wenigstens zwei unterschiedlichen Kationen und Anionen bestehen.

3. Chirale ionische Flüssigkeiten nach irgendeinem der vorangehenden Ansprüche, **dadurch gekennzeichnet, dass** das Anion [Y]ⁿ⁻ der organischen oder anorganischen Protonensäure ausgewählt ist aus der Gruppe bestehend aus, Fluorid, Chlorid, Bromid, Iodid, Nitrat, HSO₄⁻, H₂PO₄⁻,HPO₄²⁻, Bis-(trifluormethansulfon)-imidat, Tetrafluoroborat ([BF₄]⁻), Tetrachloroborat ([BCl₄]⁻), Hexafluorophosphat ([PF₆]⁻), Hexafluoroantimonat ([SbF₆]⁻), Hexafluoroarsenat ([AsF₆]⁻), Tetrachloroaluminat ([AlCl₄]⁻), Trichlorozinkat [(ZnCl₃]⁻), Dichlorocuprat, Sulfat ([SO₄]²⁻), Carbonat ([CO₃]²⁻), Fluorosulfonat, [R'-COO]⁻, [R'-SO₃]⁻ oder [(R'-SO₂)₂N]⁻, und R' ein linearer oder verzweigter 1 bis 12 Kohlenstoffatome enthaltender aliphatischer oder alicyclischer Alkyloder ein C₅-C₁₈-Aryl-, C₅-C₁₈-Aryl-C₁-C₆-alkyl- oder C₁-C₆-Alkyl-C₅-C₁₈-aryl-Rest ist, der durch Halogenatome substituiert sein kann.

4. Chirale ionische Flüssigkeiten nach Anspruch 3, **dadurch gekennzeichnet, dass** die Gruppe R' wenigstens ein Chiralitätszentrum aufweist.

5. Chirale ionische Flüssigkeiten nach irgendeinem der vorangehenden Ansprüche, **dadurch gekennzeichnet, dass** [A]⁺ ausgewählt ist aus der Gruppe bestehend aus 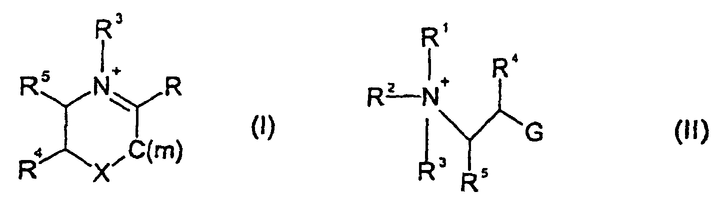 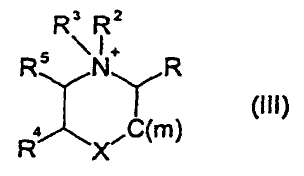 wobei
m = 0 oder 1 ist,
X aus gewählt ist aus der Gruppe bestehend aus Stickstoff, Sauerstoff und Schwefel,
R, R¹, R², R³, R⁴ und R⁵ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 30 Kohlenstoffatomen, wobei die linearen oder verzweigten aliphatischen Alkylgruppen mit einer Hydroxylgruppe substituiert sein können;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein kann;
- Aryl-, Aryl-C₁-C₆-Alkylgruppen mit 5 bis 12 Kohlenstoffatomen im Arylrest, der gegebenenfalls mit wenigstens einer C₁-C₆-Alkylgruppe und/oder einem Halogenatom substituiert sein kann, und
- Silyl-Gruppen der allgemeinen Formel -SiR⁶R⁷R⁸, wobei R⁶, R⁷ und R⁸ unabhängig voneinander ausgewählt sind aus der Gruppe bestehend aus C₁-C₆-Alkyl und C₅-C₁₂-Aryl, sowie
G ausgewählt ist aus der Gruppe bestehend aus -OH, Ether- (-OR), Thiol- (-SH), Thioether- (-SR), Nitril- (-CN), Carbonsäure- (-COOH), Carbonsäureester- (-COOR), Phosphan- (-PR₂), Keton- (-COR), Aldehyd- (-CHO), Nitro- (-NO₂), Azid- (-N₃), Phenyl-, Fluorid- oder Chlorid-, wobei, R die obengenannten Bedeutungen hat,
mit der Maßgabe, dass R⁴ und R⁵ nicht gleichzeitig Wasserstoff sind.

6. Chirale ionische Flüssigkeiten nach Anspruch 5 **dadurch gekennzeichnet, dass** R, R¹, R², R³, R⁴, R⁵ R⁶, R⁷ und R⁸ unabhängig voneinander wenigstens ein Chiralitätszentrum aufweisen können.

7. Chirale Flüssigkeit nach Anspruch 1 ausgewählt aus der Gruppe bestehend aus 4-(S)-Isopropyl-2,3-dimethyl-oxazoliniumtetrafluoroborat, 3-Butyl-4-(S)-isopropyl-2-methyl-oxazoliniumtetrafluoroborat, 3-Butyl-4-(S)-isopropyl-2-methyl-oxazolinium-hexafluorophosphat, (1-Hydroxymethyl-2-methylpropyl)-tri-methylammonium-hexafluorophosphat

8. Verfahren zur Herstellung der ionischen Flüssigkeiten nach irgendeinem der Ansprüche 1 bis 7 durch Umsetzung eines dem Ammonium-Kation [A]⁺ der allgemeinen Formel [NR^{w}R^{x}R^{y}R^{z}]⁺ zugrunde liegenden Amines NR^{x}R^{y}R^{z} mit einem Reagenz der Formel R^{w}X¹, R^{w}SO₄R^{w}, R'SO₃R^{w} oder [R^{w}₃O]⁺BF₄⁻, wobei X¹ Fluorid, Chlorid, Bromid oder Iodid ist, R^{w}, R^{x}, R^{y} und R^{z} die in Anspruch 1 angegebenen Bedeutungen haben, R^{w} jedoch nicht Wasserstoff ist, mit der Maßgabe, dass nicht mehr als zwei, vorzugsweise nicht mehr als eine der Gruppen R^{x}, R^{y} und R^{z} gleichzeitig Wasserstoff sind.

9. Verfahren zur Herstellung der ionischen Flüssigkeiten nach Anspruch 8 durch Umsetzung der optisch aktiven Amine 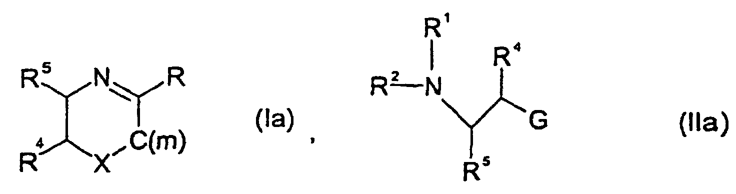 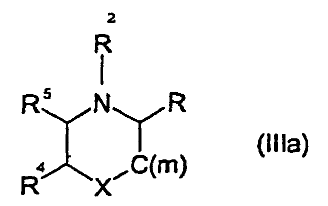 mit einem Reagenz der Formel R³X¹, R³SO₄R^{3'}, R'SO₃R³ oder [R³₃O]⁺BF₄⁻, wobei X¹ Fluorid, Chlorid, Bromid oder Iodid ist, R' die in Anspruch 4 angegebene Bedeutung hat, R³ die in Anspruch 5 angegebene Bedeutung hat und R^{3'} aus der Gruppe von R³ ausgewählt ist, jedoch von R³ verschieden sein kann.

10. Verfahren nach Anspruch 8 oder 9, **dadurch gekennzeichnet, dass** die Reaktion bei Temperaturen von -196 °C bis +150 °C durchgeführt wird.

11. Verwendung der chiralen ionischen Flüssigkeiten, wie in irgendeinem der Ansprüche 1 bis 7 definiert, zur Trennung von Racematen.

12. Verwendung der chiralen ionischen Flüssigkeiten, wie in irgendeinem der Ansprüche 1 bis 7 definiert, als Lösungsmittel für die asymmetrische Synthese.

13. Verwendung der chiralen ionischen Flüssigkeiten, wie in- irgendeinem der Ansprüche 1 bis 7 definiert, als Lösungsmittel für die asymmetrische Katalyse.

14. Verfahren zur Trennung von Enantiomerengemischen in deren Enantiomere in Gegenwart von chiralen ionischen Flüssigkeiten, wie in irgendeinem der Ansprüche 1 bis 7 definiert.

15. Verfahren nach Anspruch 14, **dadurch gekennzeichnet, dass** das Enantiomerengemisch in einem Lösungsmittel gelöst eingesetzt wird.

16. Verfahren zur Anreicherung eines Enantiomeren eines Enantiornerengemisches durch In-Kontakt-Bringen des Enantiomerengemisches mit chiralen ionischen Flüssigkeiten, wie in irgendeinem der Ansprüche 1 bis 7 definiert.

17. Verfahren nach Anspruch 16, **dadurch gekennzeichnet, dass** das Enantiomerengemisch in einem Lösungsmittel gelöst eingesetzt wird.

18. Verfahren zur asymmetrischen Synthese in Gegenwart von chiralen ionischen Flüssigkeiten, wie in irgendeinem der Ansprüche 1 bis 7 definiert.

19. Verfahren nach Anspruch 18, **dadurch gekennzeichnet, dass** die Synthese eine Diels-Alder Reaktion oder eine Benzoin-Synthese ist.

20. Verfahren zur asymmetrischen Katalyse in Gegenwart von chiralen ionischen Flüssigkeiten, wie in irgendeinem der Ansprüche 1 bis 7 definiert.

21. Verfahren nach Anspruch 20, **dadurch gekennzeichnet, dass** die Katalyse eine Hydrierung oder eine Hydroxyvinylierung ist.

## Claims

1. Chiral ionic liquids of the general formula
[A]ₙ⁺[Y]ⁿ⁻,
wherein n = 1 or 2,
the anion [Y]ⁿ⁻ is the anion of an organic or inorganic proton acid; and
the cation [A]⁺ is an optically active organic ammonium cation with up to 50 carbon atoms, wherein the ammonium cation [A]⁺ has the general formula
[NR^{w}R^{x}R^{y}R^{z}]⁺,
and
R^{w}, R^{x}, R^{y} and R^{z} are independently selected from the group comprising
- hydrogen;
- linear or branched, saturated or unsaturated, aliphatic or alicyclic alkyl groups with 1 to 30 carbon atoms;
- heteroaryl, heteroaryl C₁-C₆ alkyl groups with 3 to 8 carbon atoms in the heteroaryl radical and at least one heteroatom selected from N, O and S, which can be substituted with at least one group selected from C₁-C₆-alkyl groups and/or halogen atoms;
- aryl, aryl C₁-C₆ alkyl groups with 5 to 12 carbon atoms; and
- silyl groups of general formula -SiR^{t}R^{u}R^{v}, wherein R^{t}, R^{u} and R^{v} are independently selected from the group comprising C₁-C₆ alkyl and C₅-C₁₂ aryl;
wherein at least one of these R^{w}, R^{x}, R^{y} and R^{z} groups has at least one chirality centre;
at least one of the R^{w}, R^{x}, R^{y} and R^{z} groups is either substituted with or contains at least one functional group selected from the group comprising -OH, ether (-OR), thiol (-SH), thioether (-SR), nitrile (-CN), carboxylic acid (-COOH), carboxylic acid ester (-COOR), phosphane (-PR₂), ketone (-COR), aldehyde (-CHO), nitro (-NO₂), azide (-N₃), phenyl, fluoride or chloride, wherein R is selected from the group comprising
- hydrogen;
- linear or branched, saturated or unsaturated, aliphatic or alicyclic alkyl groups with 1 to 30 carbon atoms, wherein said linear or branched aliphatic alkyl groups may be substituted with a hydroxy group;
- heteroaryl, heteroaryl C₁-C₆ alkyl groups with 3 to 8 carbon atoms in the heteroaryl radical and at least one heteroatom selected from N, O and S, which can be substituted with at least one group selected from C₁-C₆-alkyl groups and/or halogen atoms;
- aryl, aryl C₁-C₆ alkyl groups with 5 to 12 carbon atoms in the aryl radical which may be optionally substituted with at least one C₁-C₆-alkyl group and/or a halogen atom; and
- silyl groups of general formula -SiR⁶R⁷R⁸, wherein R⁶, R⁷ and R⁸ are independently selected from the group comprising C₁-C₆ alkyl and C₅-C₁₂ aryl;
two of the R^{w}, R^{x}, R^{y} and R^{z} groups can be linked by forming a 4, 5, 6 or 7-membered saturated or unsaturated ring, which can in addition contain at least one heteroatom selected from nitrogen, oxygen or sulfur, and this ring can be substituted with at least one of the above mentioned alkyl, aryl or heteroaryl groups;
wherein at least one chirality centre has a distance of up to 5 atomic bonds from the functional group;
provided that not more than one, preferably not more than two, more preferably not more than three of the R^{w}, R^{x}, R^{y} and R^{z} groups are at the same time hydrogen.

2. The chiral ionic liquids as claimed in claim 1, **characterized by** comprising a mixture of at least two different cations and anions.

3. The chiral ionic liquids as claimed in any of the foregoing claims, **characterized in that** the anion [Y]ⁿ⁻ of the organic or inorganic proton acid is selected from the group comprising fluoride, chloride, bromide, iodide, nitrate, HSO₄⁻, H₂PO₄⁻, HPO₄²⁻, bis(trifluoromethane sulfone)imidate, tetrafluoroborate ([BF₄]⁻), tetrachloroborate ([BCl₄]⁻), hexafluorophosphate ([PF₆]⁻), hexafluoroantimonate ([SbF₆]⁻), hexafluoroarsenate ([AsF₆]⁻), tetrachloroaluminate ([AlCl₄]⁻), trichlorozincate [(ZnCl₃]⁻), dichlorocuprate, sulfate ([SO₄]²⁻), carbonate ([CO₃]²⁻), fluorosulfonate, [R'-COO]⁻, [R'-SO₃]⁻ or [(R'-SO₂)₂N]⁻, wherein R' is a linear or branched aliphatic or alicyclic alkyl containing 1 to 12 carbon atoms or C₅-C₁₈ aryl, C₅-C₁₈-aryl-C₁-C₆-alkyl or C₁-C₆-alkyl-C₅-C₁₈-aryl radical, which may be substituted with halogen atoms.

4. The chiral ionic liquids as claimed in claim 3, **characterized in that** the R' group has least one chirality centre.

5. The chiral ionic liquids as claimed in any of the foregoing claims, **characterized in that** [A]⁺ has is selected from the group comprising 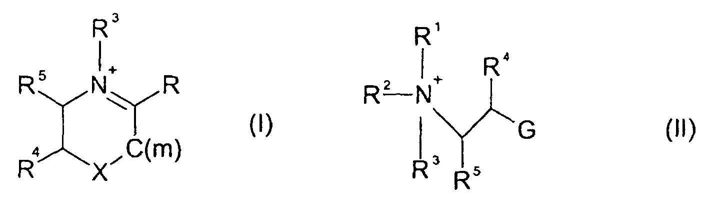 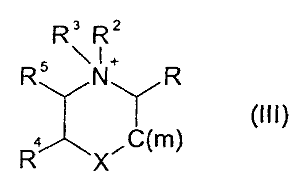 wherein
m = 0 or 1,
X is selected from the group comprising nitrogen, oxygen and sulfur;
R, R¹, R², R³, R⁴ and R⁵ are independently selected from the group comprising
- hydrogen;
- linear or branched, saturated or unsaturated, aliphatic or alicyclic alkyl groups with 1 to 30 carbon atoms, wherein the linear or branched aliphatic alkyl groups may be substituted with a hydroxy group;
- heteroaryl, heteroaryl C₁-C₆ alkyl groups with 3 to 8 carbon atoms in the heteroaryl radical and at least one heteroatom selected from N, O and S, which may be substituted with at least one group selected from C₁-C₆ alkyl groups and/or halogen atoms;
- aryl, aryl C₁-C₆ alkyl groups with 5 to 12 carbon atoms in the aryl radical, which may be optionally substituted with at least one C₁-C₆ alkyl group and/or one halogen atom; and
- silyl groups of general formula -SiR⁶R⁷R⁸, whereby R⁶, R⁷ and R⁸ are independently selected from the group comprising C₁-C₆ alkyl and C₅-C₁₂ aryl; and
G is selected from the group comprising -OH, ether (-OR), thiol (-SH), thioether (-SR), nitrile (-CN), carboxylic acid (-COOH), carboxylic acid ester (-COOR), phosphane (-PR₂), ketone (-COR), aldehyde (-CHO), nitro (-NO₂), azide (-N₃), phenyl, fluoride or chloride, wherein R has the above mentioned meanings;
provided that R⁴ and R⁵ are not simultaneously hydrogen.

6. The chiral ionic liquids as claimed in claim 5 **characterized in that** R, R¹, R², R³, R⁴, R⁵, R⁶, R⁷ and R⁸ can have at least one chirality centre independently of one another.

7. The chiral liquid as claimed in claim 1 selected from the group comprising 4-(S)-isopropyl-2,3-dimethyloxazolinium tetrafluoroborate, 3-butyl-4-(S)-isopropyl-2-methyloxazolinium tetrafluoroborate, 3-butyl-4-(S)-isopropyl-2-methyloxazolinium hexafluorophosphate, (1-hydroxymethyl-2-methyl-propyl)trimethylammonium hexafluorophosphate.

8. A method for producing the ionic liquids as claimed in any of claims 1 to 7 by converting an amine NR^{x}R^{y}R^{z} on which the ammonium cation [A]⁺ of general formula [NR^{w}R^{x}R^{y}R^{z}]⁺ is based with a reagent of formula R^{w}X¹, R^{w}SO₄R^{w}, R'SO₃R^{w} or [R^{w}₃O]⁺BF₄⁻, wherein X¹ is fluoride, chloride, bromide or iodide, R^{w}, R^{x}, R^{y} and R^{z} have the meanings given in claim 1, but R^{w} is not hydrogen, provided that not more than two, preferably not more than one, of the R^{x}, R^{y} and R^{z} groups are hydrogen at the same time.

9. The method for producing the ionic liquids as claimed in claim 8 by conversion of the optically active amines 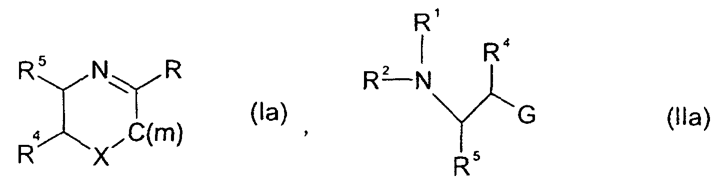 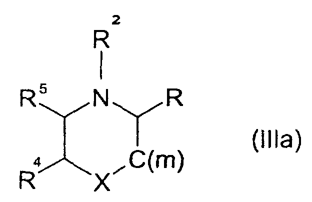 with a reagent of formula R³X¹, R³SO₄R^{3'}, R'SO₃R³ or [R³₃O]⁺BF₄⁻,
wherein X¹ is fluoride, chloride, bromide or iodide, R' has the meaning given in claim 4, R³ has the meaning given in claim 5 and R^{3'} is selected from the R³ group, but may be different from R³.

10. The method as claimed in claim 8 or 9, **characterized in that** the reaction is carried out at temperatures of -196 °C to +150 °C.

11. Use of the chiral ionic liquids as defined in any of claims 1 to 7 for the optical resolution of racemates.

12. Use of the chiral ionic liquids as defined in any of claims 1 to 7 as a solvent for asymmetric synthesis.

13. Use of the chiral ionic liquids as defined in any of claims 1 to 7 as a solvent for asymmetric catalysis.

14. A method for separating enantiomer mixtures into their enantiomers in the presence of chiral ionic liquids as defined in any of claims 1 to 7.

15. The method as claimed in claim 14, **characterized in that** the enantiomer mixture is used in a form dissolved in a solvent.

16. A method for enriching an enantiomer of an enantiomer mixture by contacting the enantiomer mixture with chiral ionic liquids as defined in any of claims 1 to 7.

17. The method as claimed in claim 16, **characterized in that** the enantiomer mixture is used in a form dissolved in a solvent.

18. A method for asymmetric synthesis in the presence of chiral ionic liquids as defined in any of claims 1 to 7.

19. The method as claimed in claim 18, **characterized in that** the synthesis is a Diels-Alder reaction or a benzoin synthesis.

20. A method for asymmetric catalysis in the presence of chiral ionic liquids as defined in any of claims 1 to 7.

21. The method as claimed in claim 20, **characterized in that** the catalysis is a hydrogenation or hydroxyvinylation.

## Revendications

1. Liquides ioniques chiraux de formule générale
[A]ₙ⁺[Y]ⁿ⁻,
où
n = 1 ou 2,
l'anion [Y]ⁿ⁻ est l'anion d'un acide protonique organique ou inorganique ; et
le cation [A]⁺ est un cation ammonium organique optiquement actif ayant jusqu'à 50 atomes de carbone, où le cation ammonium [A]⁺ possède la formule générale
[NR^{w}R^{x}R^{y}R^{z}]⁺
et
R^{w}, R^{x}, R^{y} et R^{z} sont choisis indépendamment les uns des autres dans le groupe consistant en
- l'hydrogène ;
- les groupes alkyle aliphatiques ou alicycliques saturés ou insaturés, linéaires ou ramifiés, à 1 à 30 atomes de carbone ;
- les groupes hétéroaryle, hétéroaryl-C₁-C₆-alkyle à 3 à 8 atomes de carbone dans le groupement hétéroaryle et au moins un hétéroatome choisi parmi N, O et S, qui peut être substitué par au moins un groupe choisi parmi les groupes C₁-C₆-alkyle et/ou les atomes d'halogène ;
- les groupes aryle, aryl-C₁-C₆-alkyle à 5 à 12 atomes de carbone ; et
- les groupes silyle de formule générale -SiR^{t}R^{u}R^{v} où R^{t}, R^{u} et R^{v} sont choisis indépendamment les uns des autres dans le groupe consistant en C₁-C₆-alkyle et C₅-C₁₂-aryle ;
où au moins l'un de ces groupes R^{w}, R^{x}, R^{y} et R^{z} comporte au moins un centre de chiralité ;
au moins l'un des groupes R^{w}, R^{x}, R^{y} et R^{z} est substitué par au moins un groupe fonctionnel ou contient celui-ci et ce groupe fonctionnel est choisi dans le groupe consistant en -OH, éther- (-OR), thiol- (-SH), thioéther- (-SR), nitrile- (-CN), acide carboxylique- (-COOH), ester d'acide carboxylique- (-COOR), phosphane- (-PR₂), cétone- (-COR), aldéhyde- (-CHO), nitro- (-NO₂), azide- (-N₃), phényle-, fluorure- ou chlorure-, où R est choisi dans le groupe consistant en
- l'hydrogène ;
- les groupes alkyle aliphatiques ou alicycliques saturés ou insaturés, linéaires ou ramifiée, à 1 à 30 atomes de carbone, où les groupes alkyle aliphatiques linéaires ou ramifiés peuvent être substitués par un groupe hydroxyle ;
- les groupes hétéroaryle, hétéroaryl-C₁-C₆-alkyle à 3 à 8 atomes de carbone dans le groupement hétéroaryle et au moins un hétéroatome choisi parmi N, O et S, qui peut être substitué par au moins un groupe choisi parmi les groupes C₁-C₆-alkyle et/ou les atomes d'halogène ;
- les groupes aryle, aryl-C₁-C₆-alkyle à 5 à 12 atomes de carbone dans le groupement aryle, qui peut éventuellement être substitué par au moins un groupe C₁-C₆-alkyle et/ou un atome d'halogène, et
- les groupes silyle de formule générale -SiR⁶R⁷R⁸ où R⁶, R⁷ et R⁸ sont choisis indépendamment les uns des autres dans le groupe consistant en C₁-C₆-alkyle et C₅-C₁₂-aryle ;
deux des groupes R^{w}, R^{x}, R^{y} et R^{z} peuvent être liés en formant un cycle saturé ou insaturé à 4, 5, 6 ou 7 chaînons, qui peut contenir en outre au moins un hétéroatome choisi parmi l'azote, l'oxygène ou le soufre, et ce cycle peut être substitué par au moins l'un des groupes alkyle, aryle ou hétéroaryle cités ci-dessus ;
où au moins un centre de chiralité a une distance avec le groupe fonctionnel pouvant atteindre 5 liaisons atomiques ;
avec la condition que pas plus d'un, de préférence pas plus de deux, de manière particulièrement préférée pas plus de trois des groupes R^{w}, R^{x}, R^{y} et R^{z} sont simultanément l'hydrogène.

2. Liquides ioniques chiraux selon la revendication 1 **caractérisés en ce qu'**ils consistent en un mélange d'au moins deux cations et anions différents.

3. Liquides ioniques chiraux selon l'une quelconque des revendications précédentes **caractérisés en ce que** l'anion [Y]ⁿ⁻ de l'acide protonique organique ou inorganique est choisi dans le groupe consistant en fluorure, chlorure, bromure, iodure, nitrate, HSO₄⁻, HPO₄⁻, HPO₄²⁻, bis-(trifluorométhanesulfone)imidate, tétrafluoroborate ([BF₄]⁻), tétrachloroborate ([BCl₄]⁻), hexafluorophosphate ([PF₆]⁻), hexafluoroantimonate ([SbF₆]⁻), hexanfluoroarséniate ([AsF₆]⁻), tétrachloro-aluminate ([AlCl₄]⁻), trichlorozincate ([ZnCl₃]⁻), dichlorocuprate, sulfate ([SO₄]²⁻), carbonate ([CO₃]²⁻), fluorosulfonate, [R'-COO]⁻, [R'-SO₃]⁻ ou [(R'-SO₂]₂N]⁻ , et R' est un groupement alkyle aliphatique ou alicyclique linéaire ou ramifié contenant 1 à 12 atomes de carbone ou un groupement C₅-C₁₈-aryle, C₅-C₁₈-aryl-C₁-C₆-alkyle ou C₁-C₆-alkyl-C₅-C₁₈-aryle qui peut être substitué par des atomes d'halogène.

4. Liquides ioniques chiraux selon la revendication 3 **caractérisés en ce que** le groupe R' comporte au moins un centre de chiralité.

5. Liquides ioniques chiraux selon l'une quelconque des revendications précédentes **caractérisés en ce que** [A]⁺ est choisi dans le groupe consistant en 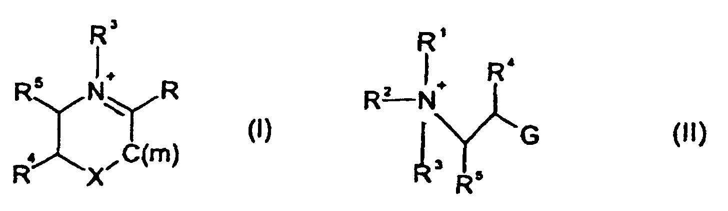 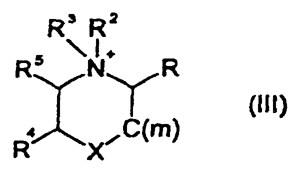 où
m = 0 ou 1,
X est choisi dans le groupe consistant en l'azote, l'oxygène et le soufre,
R, R¹, R², R³, R⁴ et R⁵ sont choisis indépendamment les uns des autres dans le groupe consistant en
- l'hydrogène ;
- les groupes alkyle aliphatiques ou alicycliques saturés ou insaturés, linéaires ou ramifiée, à 1 à 30 atomes de carbone, où les groupes alkyle aliphatiques linéaires ou ramifiés peuvent être substitués par un groupe hydroxyle ;
- les groupes hétéroaryle, hétéroaryl-C₁-C₆-alkyle à 3 à 8 atomes de carbone dans le groupement hétéroaryle et au moins un hétéroatome choisi parmi N, O et S, qui peut être substitué par au moins un groupe choisi parmi les groupes C₁-C₆-alkyle et/ou les atomes d'halogène ;
- les groupes aryle, aryl-C₁-C₆-alkyle à 5 à 12 atomes de carbone dans le groupement aryle, qui peut éventuellement être substitué par au moins un groupe C₁-C₆-alkyle et/ou un atome d'halogène, et
- les groupes silyle de formule générale -SiR⁶R⁷R⁸ où R⁶, R⁷ et R⁸ sont choisis indépendamment les uns des autres dans le groupe consistant en C₁-C₆-alkyle et C₅-C₁₂-aryle, et
G est choisi dans le groupe consistant en -OH, éther- (-OR), thiol- (-SH), thioéther- (-SR), nitrile- (-CN), acide carboxylique- (-COOH), ester d'acide carboxylique- (-COOR), phosphane- (-PR₂), cétone- (-COR), aldéhyde- (-CHO), nitro- (-NO₂), azide- (-N₃), phényle-, fluorure- ou chlorure-, où R a les significations citées ci-dessus,
avec la condition que R⁴ et R⁵ ne sont pas simultanément l'hydrogène.

6. Liquides ioniques chiraux selon la revendication 5 **caractérisés en ce que** R, R¹, R², R³, R⁴, R⁵, R⁶, R⁷ et R⁸ peuvent comporter indépendamment les uns des autres au moins un centre de chiralité.

7. Liquide chiral selon la revendication 1 choisi dans le groupe consistant en le tétrafluoroborate de 4-(S)-isopropyl-2,3-diméthyl-oxazolinium, le tétrafluoroborate de 3-butyl-4-(S)-isopropyl-2-méthyl-oxazolinium, l'hexafluorophosphate de 3-butyl-4-(S)-isopropyl-2-méthyl-oxazolinium, l'hexafluorophosphate de (1-hydroxyméthyl-2-méthylpropyl)-tri-méthylammonium.

8. Procédé de préparation des liquides ioniques selon l'une quelconque des revendications 1 à 7 par réaction d'une amine NR^{x}R^{y}R^{z} à la base du cation ammonium [A]⁺ de formule générale NR^{w}R^{x}R^{y}R^{z}]⁺ avec un réactif de formule R^{w}X¹, R^{w}SO₄R^{w}, R'SO₃R^{w} ou [R^{w}₃O]⁺BF₄⁻, où X¹ est fluorure, chlorure, bromure ou iodure, R^{w}, R^{x}, R^{y} et R^{z} ont les significations indiquées dans la revendication 1, cependant R^{w} n'est pas l'hydrogène,
avec la condition que pas plus de deux, de préférence pas plus d'un des groupes R^{x}, R^{y} et R^{z} sont en même temps l'hydrogène.

9. Procédé de préparation des liquides ioniques selon la revendication 8 par réaction des amines optiquement actives 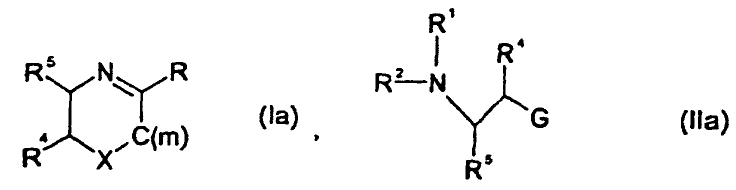 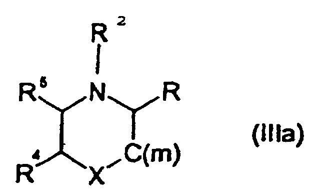 avec un réactif de formule R³X¹, R³SO₄R^{3'}, R'SO₃R³ ou [R³₃O]⁺BF₄⁻, où X¹ est fluorure, chlorure, bromure ou iodure, R' a la signification indiquée dans la revendication 4, R³ a la signification indiquée dans la revendication 5 et R^{3'} est choisi dans le groupe de R³, mais peut être différent de R³.

10. Procédé selon la revendication 8 ou 9 **caractérisé en ce que** la réaction est conduite à des températures de -196°C à + 150°C.

11. Utilisation des liquides ioniques chiraux, tels que définis dans l'une quelconque des revendications 1 à 7, pour la séparation de racémates.

12. Utilisation des liquides ioniques chiraux, tels que définis dans l'une quelconque des revendications 1 à 7, comme solvant pour la synthèse asymétrique.

13. Utilisation des liquides ioniques chiraux, tels que définis dans l'une quelconque des revendications 1 à 7, comme solvant pour la catalyse asymétrique.

14. Procédé de résolution de mélanges d'énantiomères en leurs énantiomères en présence de liquides ioniques chiraux tels que définis dans l'une quelconque des revendications 1 à 7.

15. Procédé selon la revendication 14 **caractérisé en ce que** le mélange d'énantiomères est utilisé dissous dans un solvant.

16. Procédé pour enrichir un énantiomère d'un mélange d'énantiomères par mise en contact du mélange d'énantiomères avec des liquides ioniques chiraux tels que définis dans l'une quelconque des revendications 1 à 7.

17. Procédé selon la revendication 16 **caractérisé en ce que** le mélange d'énantiomères est utilisé dissous dans un solvant.

18. Procédé de synthèse asymétrique en présence de liquides ioniques chiraux tels que définis dans l'une quelconque des revendications 1 à 7.

19. Procédé selon la revendication 18 **caractérisé en ce que** la synthèse est une réaction de Diels-Alder ou une synthèse de benzoïne.

20. Procédé de catalyse asymétrique en présence de liquides ioniques chiraux tels que définis dans l'une quelconque des revendications 1 à 7.

21. Procédé selon la revendication 20 **caractérisé en ce que** la catalyse est une hydrogénation ou une hydroxyvinylation.
